# EUROPEAN PATENT SPECIFICATION

(11) **EP 0 532 109 B1**
(45) Date of publication and mention of the grant of the patent: **08.01.1997**
(21) Application number: 92202725.5
(22) Date of filing: 08.09.1992
(51) Int. Cl.: A61M 25/00, A61M 25/01

(54) **Double lumen catheter**
Doppellumiger Katheter
Catheter à double lumière

(30) Priority: 10.09.1991 NL 9101534
(43) Date of publication of application: 17.03.1993
(73) Proprietor: CORDIS EUROPA N.V., NL-9301 LJ Roden (NL)
(72) Inventor: Boudewijn, Alexander Christiaan, NL-9351 LA Leek (NL); Bosma, Gjalt, NL-9201 EK Drachten (NL)
(74) Representative: 't Jong, Bastiaan Jacobus

(56) References cited:
- EP-A- 0 200 919
- EP-A- 0 266 928
- EP-A- 0 310 224
- EP-A- 0 321 614
- EP-A- 0 366 794
- CA-A- 1 219 785
- US-A- 3 757 768
- US-A- 4 619 643
- US-A- 5 040 548

## Description

The invention relates to a method for manufacturing a double-lumen catheter. From CA-A-1 219 785 a method for manufacturing such a catheter is known. According to this known method two single lumen catheter parts are connected to a proximal end of a double lumen catheter part. The double lumen catheter part is firstly heat treated around mandrels, such that the end parts of these lumens are widened to take up end parts of the single lumen catheter parts.

The present invention has for its object to provide a method for manufacturing a catheter of the kind set forth above, wherein it is assured that the transition in the respective lumens is smooth, so that guide wires or optical fibers can be guided through the lumens, without any obstruction.

This object is achieved with the method according to the invention as claimed in claim 1.

Achieved with the method according to the invention is that the transition from the single-channel catheter part to the fork piece and from the fork piece to the two-channel catheter part is uniform, without internally protruding edges which could make the introduction of the guide wire and/or fibre bundle difficult.

From US-A-4 619 643 the use of a molded Y-piece is known as such. A smooth transition from the cross sectional shape of the lumens in the single lumen tube and the double lumen tube is not shown in this specification.

A further favorable development is characterized in claim 2. By thus giving the core wires a diameter slightly larger than the channels, the material is slightly expanded when the core wires are inserted so that a close-fitting engagement is obtained on the core wires. A very smooth transition is thus obtained after the injection molding of the fork piece.

The step of claim 3 is preferably applied. When the core wires are pulled out of the formed fork piece, they deform elastically when following the channel formed in the fork piece by the first portion of the core wire, so that the core wires can be taken out easily. A suitable embodiment enabling repeated use is therein characterized in claim 4.

With the method according to claim 5 it is possible to feed flushing liquid to the free end of the catheter so that the blood at that location will be flushed away and the wall of the blood vessel can be examined as soon as the optic fibre bundle is pushed out of the end of the catheter.

The invention will be further elucidated in the following description with reference to the annexed figures.

Fig. 1 shows a perspective view of a piece of two-channel catheter basic material which is used in the method according to the present invention.

Fig. 2 shows on a smaller scale a second step of the embodiment of the method.

Fig. 3 shows a third step of the embodiment of the method.

Fig. 4 shows a simplified top view of a double-lumen catheter manufactured with the method according to the invention.

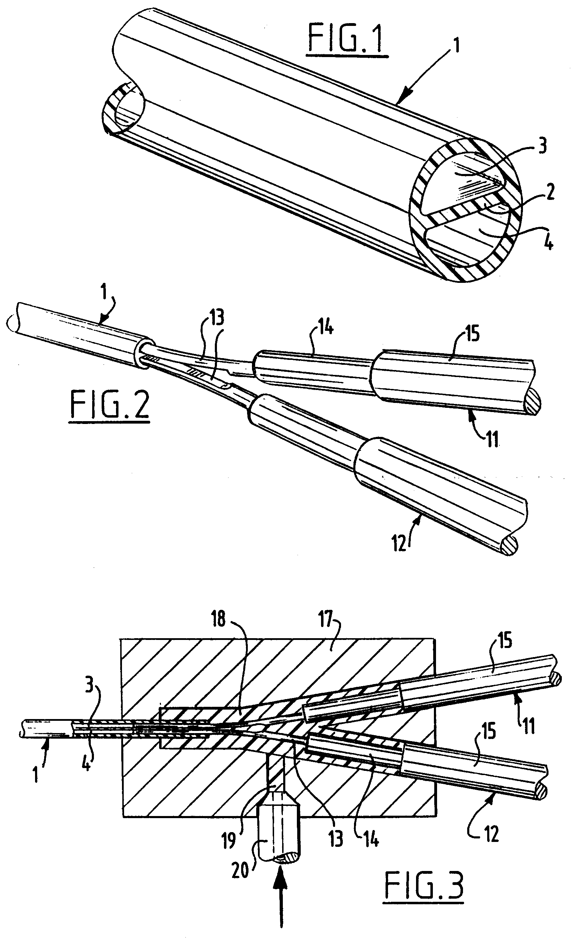

The starting material in applying the method is a piece of tube-like catheter basic material 1, such as that shown in fig. 1, with two parallel channels 3 and 4 mutually separated by a dividing wall 2. These channels have a D-shaped section, as can be seen in fig. 1.

In the method according to the invention core wires 11, 12 are inserted into the end parts of the channels 3, 4 as shown in fig. 3. Each of these core wires 11, 12 comprises a first portion 13 with a diameter which is slightly, for example 0.1 mm, larger than the diameter of the channels 3, 4 so that these first portions 13 of the core wires 11, 12 slightly expand the basic material when inserted.

Each core wire 11, 12 further comprises a second portion 14 having a diameter substantially equal to a piece of single-channel basic material to be described hereinafter which will form a continuous connection with the relevant lengthwise channel of the two-channel basic material 1.

Situated between the first portion 13 and the second portion 14 is a transitional portion the sectional shape of which gradually transposes from that of the first portion to that of the lengthwise channel of that single-channel basic material.

Each core wire 11, 12 moreover has an end part 15 which is intended for the fixing in a mould.

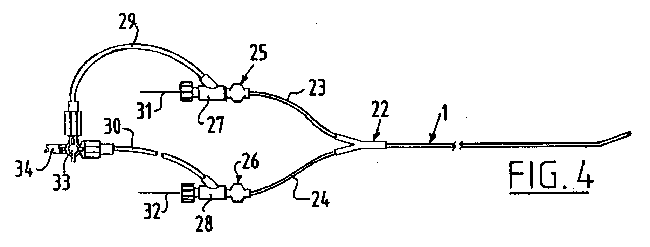

This mould 17 is shown schematically in fig. 3. It can be seen that a Y-shaped mould cavity 18 has been recessed into the mould 17. The end part of the basic material 1 is received close to the stem of the Y with the first portions of the core wires 11, 12 inserted into the channels 3, 4. The fixing parts 15 of the core wires 11, 12 are clamped in the extensions of the arms of the Y. As can be seen clearly from fig. 3, the mould cavity 18 defines a fork piece as designated in fig. 4 by 22 wherein a channel connecting precisely onto the end parts of the channels 3, 4 is recessed by the first portions 13 of the core wires 11, 12, which channel transposes into a wider portion which is defined by the second portions 14 and into which pieces of single-channel catheter basic material 23, 24 (fig. 4) are fixed, for example glued fixedly. Owing to the transition defined by the transitional portion of the core wires the channels of the single-channel parts 23, 24 connect precisely and without abrupt transition onto the channels in the fork piece 22 recessed by the first portions 13 of the core wires 11, 12.

The mould 17 is further provided with a feed channel 19 onto which a nozzle 20 of an injection molding machine 20 can connect. A suitable plastic is injected via this nozzle 20 into the mould cavity 18. After curing, this plastic forms the fork piece 22.

The end parts 13 of the core wires 11, 12 are preferably of a flexible material, preferably spring steel. The end wires can hereby easily be removed after the formed assembly has been taken out of the mould 17. After removal of the core wires 11, 12 the above-mentioned single-channel catheter material 23, 24 is glued into the fork piece 22.

Per se known connecting members 25, 26 are fastened, for example fixedly glued, to the other ends of the pieces of single-channel catheter material 23, 24.

The double-channel part of the thus obtained double-lumen catheter can be inserted in a patient. An optic fibre bundle 31 of an endoscope can be introduced via the one channel that is accessible via the connecting member 25. For this purpose the connecting member 25 is provided with a fitting element 27 which is generally known as a haemostasis valve and which has an axial passage for the fibre bundle 31 provided with a sealing co-acting with the fibre bundle 31 and a side channel. Joined to this side channel is a tube 29 which can be connected to a valve 33 which is joined via a conduit 34 to a flushing liquid source.

In similar manner a guide wire 32 can be pushed into the other channel of the double-channel catheter part. The single channel catheter part 24 joined in the fork piece 22 to the relevant lengthwise channel of the basic material 1 ends in a corresponding manner in a connecting member 26 which can likewise be provided with a valve 28 which has an axial channel for the guide wire 32 and a side channel, which is likewise connected via a tube 30 to the flushing liquid source.

Owing to the uniform transition obtained with the method between both channels of the two-channel catheter part 1 and the single channel catheter parts 23, 24 the insertion of the optic fibre bundle 31 and the guide wire 32 can be performed with no problem without irritation being caused during insertion by protruding edges or steps in the fork piece.

During use flushing liquid is supplied via the channel of the guide wire, that is, via tube 30, whereby the blood vessel at the leading end of the catheter becomes free of blood.

With the thus obtained catheter the guide wire therefore remains in the catheter throughout the entire procedure and does not have to be exchanged for the endoscope fibre bundle. The guide wire can moreover be used therefore for operating the end part of the catheter in a controlled manner in order to control the viewing direction of the endoscope fibre bundle. The guide wire lumen can moreover be used as supply channel for flushing liquid so that it is possible to simultaneously manipulate the catheter and look through the endoscope.

## Claims

1. Method for manufacturing a double-lumen catheter, comprising
providing a piece (1) of tube-like catheter basic material with two parallel, mutually separated lengthwise channels,
providing two pieces (23, 24) of tube-like catheter basic material each having one lengthwise channel
providing two core wires (11, 12), each with a first portion (13) closely fitting in the channels of the two-channel basic material (1), a second portion (14) with a cross section substantially corresponding with the external cross section of the single-channel basic material (23, 24) and a transitional portion therebetween, the shape of which gradually transposes from that of the first portion to that of the lengthwise channel of the single-channel basic material,
inserting the first portion (13) of each core wire (11, 12) into each channel (3, 4) in the end part of the two-channel basic material (1),
providing a mould (17) with a Y-shaped mould cavity (18), which is provided with fixing means for enclosed receiving of the end part (15) of the two-channel basic material close to the foot of the Y and with fixing means for enclosed receiving of the second portion of the core wires close to the ends of the arms of the Y,
receiving into the mould (17) the end part of the two-channel basic material (1) with the core wires (11, 12) inserted therein,
filling the mould cavity (18) with a curable material,
removing the formed assembly from the mould cavity (18) after the curable material has hardened to a fork piece (22),
removing the core wires (11, 12) from the fork piece (22),
fixing the pieces (23, 24) of single-channel basic material into the cavities in the fork piece (22) defined by the second portions (14) of the core wires (11, 12),
fixing to the other ends of the pieces (23, 24) of single-channel basic material connecting members (25, 26) which can allow passage respectively of a guide wire (32) and an optic fibre bundle (31).

2. Method as claimed in claim 1, wherein core wires (11, 12) are selected in which the first portion has a diameter substantially 0.1 mm larger than the lengthwise channels (3, 4) of the two-channel basic material (1).

3. Method as claimed in any of the preceding claims, wherein core wires (11, 12) are selected in which the first portion (13) and the transitional portion are elastically flexible.

4. Method as claimed in any of the preceding claims, wherein core wires (11, 12) are selected in which the first portion (13) and the transitional portion are manufactured from spring steel.

5. Method as claimed in any of the preceding claims, wherein at least for the connecting member for passage of the guide wire (32), a connecting member (26, 28) is chosen having an axial passage for the guide wire (32), a sealing which sealingly grips the guide wire (32) close to the open end of the passage and with a side channel (30) connected to the passage and joined to a supply conduit (34) for flushing liquid.

## Patentansprüche

1. Verfahren zum Herstellen eines doppellumigen Katheders mit
Vorsehen eines Stückes (1) eines röhrenförmigen Kathederbasismateriales mit zwei parallelen, gegenseitig voneinander getrennten Längskanälen,
Vorsehen zweier Stücke (23, 24) eines röhrenförmigen Kathederbasismateriales, von denen jedes einen Längskanal aufweist,
Vorsehen zweier Kerndrähte (11, 12), jeder mit einem ersten Abschnitt (13), der eng in die Kanäle des zweikanaligen Basismateriales gepaßt ist, und einem zweiten Abschnitt (14) mit einem Querschnitt, der im wesentlichen dem äußeren Querschnitt des einkanaligen Basismateriales (23, 34) entspricht, und einem Übergangsabschnitt dazwischen, dessen Form allmählich von der des ersten Abschnittes zu der des Längskanales des einkanaligen Basismateriales übergeht.
Einführen des ersten Abschnittes (13) eines jeden Kerndrahtes (11, 12) in jeden Kanal (3, 4) in dem Endteil des zweikanaligen Basismateriales (1),
Vorsehen einer Form (17) mit einem Y-förmigen Formenhohlraum (18), der mit einem Befestigungsmittel zum eingeschlossenen Aufnehmen des Endteiles (15) (des zweikanaligen Basismateriales nahe dem Fuß des Y und mit einem Befestigungsmittel zum eingeschlossenen Aufnehmen des zweiten Abschnittes der Kerndrähte nahe den Enden der Arme des Y versehen ist,
Aufnehmen des Endteiles des zweikanaligen Basismateriales (1) mit den darin eingeführten Kerndrähten (11, 12) in der Form (17),
Füllen des Formhohlraumes (18) mit einem härtbaren Material,
Entnehmen der geformten Anordnung aus dem Formenhohlraum (18), nachdem das härtbare Material zu einem Gabelstück (22) gehärtet ist,
Entnehmen der Kerndrähte (11, 12) aus dem Gabelstück (22),
Befestigen der Stücke (23, 24) des einkanaligen Basisimateriales in den Hohlräumen des Gabelstückes (22), die durch die zweiten Abschnitte (14) der Kerndrähte (11, 12) definiert werden,
Befestigen von Verbindungsteilen (25, 26), die den Durchgang eines Führungsdrahtes (32) bzw. eines optischen Faserbündels (31) erlauben können, an den anderen Enden der Stücke (23, 24) des einkanaligen Basismateriales

2. Verfahren nach Anspruch 1, bei dem Kerndrähte (11, 12) ausgewählt werden, bei denen der erste Abschnitt einen Durchmesser im wesentlichen von 0,1 mm größer als die Längskanäle (3, 4) des zweikanaligen Basismateriales (1) aufweist.

3. Verfahren nach einem der vorhergehenden Ansprüche, bei dem Kerndrähte (11, 12) ausgewählt werden, bei denen der erste Abschnitt (13) und der Übergangsabschnitt elastisch flexibel sind.

4. Verfahren nach einem der vorhergehenden Ansprüche, bei dem Kerndrähte (11, 12) ausgewählt werden, bei denen der erste Abschnitt (13) und der Übergangsabschnitt aus Federstahl hergestellt werden.

5. Verfahren nach einem der vorhergehenden Ansprüche, bei dem mindestens für das Verbindungsteil für den Durchgang des Führungsdrahtes (32) ein Verbindungsteil (26, 28) gewählt wird mit einem axialen Durchgang für den Führungsdraht (32), einer Abdichtung, die dichtend den Führungsdraht (32) nahe dem offenen Ende des Durchganges greift, und mit einem Seitenkanal (30), der mit dem Durchgang verbunden ist und mit einer Lieferleitung (34) für Spülflüssigkeit verbunden ist.

## Revendications

1. Procédé pour réaliser un cathéter à double lumière, comprenant les étapes consistant à :
- réaliser un morceau (1) de matériau de base de cathéter tubulaire comportant deux canaux longitudinaux parallèles, séparés l'un de l'autre,
- réaliser deux morceaux (23, 24) de matériau de base de cathéter tubulaire, comportant tous deux un canal longitudinal unique,
- réaliser deux fils d'âme (11, 12), comportant tous deux une première partie (13) ajustée étroitement dans les canaux du matériau de base (1) à deux canaux, une seconde partie (14) dont la section correspond sensiblement à la section extérieure du matériau de base (23, 24) à canal unique, et une partie de transition entre celles-ci, dont la forme passe progressivement de celle de la première partie à celle du canal longitudinal du matériau de base à canal unique,
- insérer la première partie (13) de chaque fil d'âme (11, 12) à l'intérieur de chaque canal (3, 4) formé dans la partie d'extrémité du matériau de base (1) à deux canaux,
- réaliser un moule (17) présentant une cavité (18) de moule en forme de Y, qui comporte des moyens de fixation pour recevoir et refermer la partie d'extrémité (15) du matériau de base à deux canaux à proximité du pied du Y, et des moyens de fixation pour recevoir et refermer seconde partie des fils d'âme à proximité des extrémités des bras du Y,
- recevoir dans le moule (17) la partie d'extrémité du matériau de base (1) à deux canaux avec les fils d'âme (11, 12) qui y sont insérés,
- remplir la cavité (18) de moule avec une matière durcissable,
- démouler de la cavité (18) l'ensemble formé, après que la matière durcissable s'est durcie sous forme d'une partie (22) en forme de fourche,
- retirer les fils d'âme (11, 12) de la partie (22) en forme de fourche,
- fixer les parties (23, 24) de matériau de base à canal unique à l'intérieur des cavités de la portion (22) en forme de fourche délimitée par les secondes parties (14) des fils d'âme (11, 12),
- fixer aux autres extrémités des parties (23, 24) de matériau de base à canal unique, des éléments (25, 26) de raccordement qui peuvent permettre le passage respectivement d'un fil de guidage (32) et d'un faisceau (31) de fibres optiques.

2. Procédé selon la revendication 1, dans lequel les fils d'âme (11, 12) sont choisis de telle sorte que la première partie a un diamètre plus grand, d' à peu près 0,1 mm, que celui des canaux longitudinaux (3, 4) du matériau de base (1) à deux canaux.

3. Procédé selon l'une quelconque des revendications précédentes, dans lequel les fils d'âme (11, 12) sont choisis de telle sorte que la première partie (13) et la partie de transition sont élastiquement flexibles.

4. Procédé selon l'une quelconque des revendications précédentes, dans lequel les fils d'âme (11, 12) sont choisis de telle sorte que la première partie (13) et la partie de transition sont fabriqué à partir d'acier à ressorts.

5. Procédé selon l'une quelconque des revendications précédentes, dans lequel au moins pour l'élément de raccordement permettant le passage du fil de guidage (32), un élément de raccordement (26, 28) est choisi qui comporte un passage axial destiné au fil de guidage (32), un dispositif d'étanchéité qui serre de manière étanche le fil de guidage (32) près de l'extrémité ouverte du passage, et avec un canal latéral (30) raccordé au passage et raccordé à un conduit d'alimentation (34) destiné au liquide de chasse.
